# EUROPEAN PATENT APPLICATION

(11) **EP 2 368 968 A1**
(43) Date of publication of application: **28.09.2011**
(21) Application number: 10157181.8
(22) Date of filing: 22.03.2010
(51) Int. Cl.: C10G 3/00, C10L 1/06, C07C 9/14

(54) **Solvent composition**

(71) Applicant: Neste Oil Oyj, 02150 Espoo (FI)
(72) Inventor: Makkonen, Jaana, FI-01150, Söderkulla (FI); Karjalainen, Paula, FI-00730, Helsinki (FI); Rantanen-Kolehmainen, Leena, FI-06450, Porvoo (FI)
(74) Representative: Kurra, Sirpa Eliina

(57) **Abstract**

The invention relates to a solvent composition comprising 25 to 50 % of C₅-C₇ n-alkanes, and 50 to 75 % of C₅-C₇ iso-alkanes, the amount of n-hexanes being 5 to 25 %, respectively by weight, said solvent composition being produced from raw materials of biological origin.

## Description

### Field of the invention

The invention relates to solvent compositions of biological origin, containing paraffinic C5-C7 hydrocarbons, said compositions being useful for instance as technochemical and industrial solvents, drilling fluids for oil recovery, hydraulic oils, cable oils, heat transfer fluids, agricultural chemicals, fuels and as fuel components, in fuel additives, as fuels for camp cookers, fuels for heating and lightning purposes, in chemical mixtures and formulations, as a cleaning agent, as a propellant, in paints and surface treatment agents, in construction materials, in agricultural chemicals, in the production and processing of polymers, and in extraction processes.

### Prior art

At present, direct or indirect reduction of detrimental impacts on nature is aimed at in nearly all technical fields. Reduction of carbon dioxide emissions and other greenhouse gasses is the future objective.

US 2007260102 discloses solvents produced from starting materials of biological origin. In this publication, the production of n-paraffins by hydrogenation from vegetable oil is described, yielding C₁₀-C₁₃ n-paraffins useful as biological solvents.

WO 2007068800 discloses the oligomerization of carboxylic acids of biological origin to give compounds with longer chains, followed by hydrodeoxygenation thereof to obtain n-paraffins. These compounds may then be optionally isomerized to give corresponding branched iso-paraffins. C₅-C₁₀ paraffinic fraction is obtained as the product, useful as a solvent.

### Object of the invention

The object of the invention is to provide a carbon dioxide neutral solvent composition contributing to the reduction of carbon dioxide and greenhouse emissions, said composition comprising paraffinic hydrocarbons and having a lower n-hexane content than in similar solvents from fossil raw materials, typically found on the market.

Another object of the invention is also a solvent composition containing paraffinic hydrocarbons of biological origin wherein the carbon dioxide generated from the solvent during combustion thereof, or carbon dioxide sources released therefrom only contain carbon from organic material.

Characteristic features of the inventive solvent composition are presented in the protective claims.

### Definitions

The term "biosolvent" as used here refers to a solvent or a solvent composition produced from starting materials of biological origin.

### General description of the invention

The present invention is directed to an aliphatic solvent composition comprising C₅-C₇ n-alkanes and isoalkanes, the amount of n-hexane typically being from 5 to 25 %, by weight. The n-hexane content of the solvent is clearly lower than that of similar solvents from fossil raw materials, typically found on the market.

The solvent composition is produced from raw materials of biological origin, and thus said solvent composition is excellently useful as an environmentally friendly biosolvent. Carbon dioxide generated from the solvent during combustion, or carbon dioxide sources released therefrom only contain carbon derived from organic material.

The solvent composition typically has a boiling range of 35 to 150 °C, density at +15 °C of 660 to 680 kg/m³, sulfur content of up to 1 mg/kg, and a vapour pressure of 35 to 50 kPa, said composition containing no more than 0.01 %, by volume, of aromatic compounds. The solvent composition is colourless, odourless, and chemically very stable.

The solvent composition is useful for instance in technochemical and industrial applications and may be used as such, or as a mixture with other solvents and/or additives/aids used in the field. Moreover, solvents, drilling fluids for oil recovery, hydraulic oils, cable oils, heat transferring fluids, agricultural chemicals, fuels and fuel components, such as fuels for camp cookers, fuels for heating and lightning purposes, chemical mixtures and formulations, cleaning agents, propellants, paints and surface treatment agents, construction materials, and use in the production and processing of polymers, and in extraction processes may also be mentioned as examples of these applications.

### Detailed description of the invention

The inventors of this solvent composition found out that a solvent composition may be produced from n-alkanes and iso-alkanes of biological origin, which has several advantages over solvents of the prior art. The aliphatic solvent composition of the invention contains C₅-C₇ n-alkanes and iso-alkanes. More specifically, said solvent composition contains 25 to 50 % of C₅-C₇ n-alkanes and 50 to 75 % of C₅-C₇ iso-alkanes, the amount, or content, of n-hexane being 5 to 25 %, all percentages being by weight. Preferably the solvent composition comprises 35 to 45 % of C₅-C₇ n-alkanes and 55 to 70 % of C₅-C₇ iso-alkanes, the amount of n-hexane being 15 to 23 %, the amount of n-pentane being 4-8 %, and the amount of n-heptane being 11-14 %, by weight.

The solvent composition typically has a boiling range of 35 to 150 °C, density at +15 °C of 660 to 680 kg/m³, freezing point of less than -60°C, sulfur content of up to 1 mg/kg, and a vapour pressure of 35 to 50 kPa, said composition containing aromatic compounds no more than 0.01 %, by volume. The value of latent heat (LHV) of the composition is typically 44 to 45 MJ/kg. The solvent composition is colourless, odourless, and chemically very stable.

The table 1 below shows typical properties of the biosolvent composition of the invention, and the methods used for the determination thereof.

**Table 1**

| Property | Unit | Typical | Method |
|---|---|---|---|
| Aspect, +25 °C | - | clear | D 4176-1 |
| Sulfur | mg/kg | max 1.0 | EN ISO 20846 |
| Distillation, % evaporation, by vol. | °C | | EN ISO 3405 |
| IBP | %, by vol | min 35°C | EN ISO 3405 |
| FBP/EP | %, by vol | max 150 °C | EN ISO 3405 |
| Density, 15 °C | kg/m³ | 660...680 | EN ISO 12185 |
| Latent heat value LHV | MJ/kg | 44......45 | ASTM D 240 |
| Freezing point | °C | less than - 60 | EEC A1/A2 |

The composition of the invention may be produced from starting materials of biological origin using for instance a process first comprising a hydrodeoxygenation (HDO) step for decomposing the structure of the biological ester or triglyceride constituent, and for removing oxygen, phosphorus and sulfur compounds, concurrently hydrogenating the olefinic bonds, followed by isomerization of the product thus obtained, thus branching the hydrocarbon chain and improving the low temperature properties of the paraffin. The product may for instance fractionated to give the desired fractions.

Biological raw materials from plants, animals or fish containing fatty acids and/or fatty acid esters may be used as the starting material. The raw material may be selected from the group consisting of vegetable oils, animal fats, fish oils, and mixtures thereof. Suitable biological raw materials include rapeseed oil, canola oil, colza oil, tall oil, sunflower oil, soybeen oil, hemp oil, oilve oil, linenseed oil, mustard oil, palm oil, arachis oil, castor oil, coconut oil, animal fats such as suet, tallow, blubber, recycled alimentary fats, starting materials produced by genetic engineering, and biological starting materials produced by microbes such as algae and bacteria. Condensation products, esters, or other derivatives obtained from biological raw materials may also be used as starting materials.

In the HDO step, hydrogen gas and the biological constituent are passed to the HDO catalyst bed either in countercurrent or concurrent manner. In the HDO step, the pressure and the temperature range between 20 and 150 bar, and between 200 and 500 °C, respectively. In the HDO step, known hydrodeoxygenation catalysts may be used.

Prior to the HDO step, the biological raw material may optionally be subjected to prehydrogenation under milder conditions to avoid side reactions of the double bonds.

After the HDO step, the product is passed to the isomerization step where hydrogen gas and the biological constituent to be hydrogenated, and optionally the n-paraffin mixture are passed to the isomerization catalyst bed either concurrent or countercurrent manner.

In the isomerization step, the pressure and the temperature range between 20 and 150 bar, and between 200 and 500 °C, respectively. In the isomerization step, isomerization catalysts known as such may be typically used.

The solvent composition is produced from raw materials of biological origin, and accordingly, the solvent composition is very useful as a biosolvent. Since no fossil starting materials, but on the contrary, raw materials of biological origin from renewable resources are used in the production of the solvent composition of the invention, the total impact of the production and use on the greenhouse gas balance is lower than that of solvent compositions made of fossil raw materials. Greenhouse emissions of the solvent composition during the whole life cycle are typically lower than those of similar solvent compositions made of conventional fossil raw materials. Carbon dioxide generated from the solvent composition during combustion thereof, or carbon dioxide sources released therefrom only contain carbon from organic material.

The solvent composition comprises minute amounts of naphthenic compounds, typically less than 2 %, by weight. The odour of the product is thus very mild compared to that of a corresponding solvent based on crude oil, comprising about 20 %, by weight, of naphthenes. The composition typically comprises clearly less than 0.01 % of aromatic compounds, less than 0.005 % of benzene, by weight, and insignificant amounts of olefinic compounds or other reactive constituents.

The composition is chemically very stable, has a narrow boiling range, is highly compatible with various other materials, and non-corrosive with respect to metals, further, has a strong lowering effect on viscosity, and a low freezing point.

Content of n-hexanes being 5 to 25 %, by weight, that is, considerably lower than that of fossil solvent composition, the solvent composition of the invention has advantages with respect to both working safety and low temperature properties.

The solvent composition of the invention is a light and readily volatile solvent with excellent dissolving and low temperature properties, and is thus useful in various applications as such or in combination with conventional solvents. Especially the paraffinic combination of C₅-C₇ n-alkanes and iso-alkanes, with a content of n-hexanes typically lower than that of similar solvents currently in use, results in excellent properties of the solvent. A relatively narrow boiling range and high paraffinic content, the majority of which consists of iso-paraffins, in combination with the chemical stability, chemical inertia, low viscosity, and density make the composition particularly useful in several application.

Harmful impacts of conventional solvents may be reduced and the proportion of constituents of biologcical origin may be increased by mixing the solvent composition with conventional solvents.

The solvent composition of the invention is useful in various technochemical and industrial applications, including solvents, drilling fluids for oil recovery, hydraulic oils, cable oils, heat transfer fluids, agricultural chemicals, fuels and as fuel components, fuels for heating and lightning purposes, fuel additives, chemical mixtures and formulation, cleaning agents, propellants, paints and surface treatment agents, construction materials, solvents and additives used in the production of polymers, applications of mining industry, processing of polymers, and extraction processes.

The solvent composition is particularly suitable for extraction processes, as a readily volatile solvent for paints for road marking, and as washing solvents.

Moreover, as examples of solvent applications, odourless coatings and paints, paints requiring rapid evaporation, purified gasoline, propellants for spary paints, fuels for camp cookers, adhesives, non-aqueous cleaning agents, applications as plant protecting agents, dry cleaning solvents, treatment agents for wood surfaces, and wood oils, cleaning and defatting agents for industrial apparatuses and for metals in general, extracting agents to be used in industrial closed circuits, aids for machining, printing inks, aids for paper production, process fluids, cleaning and polishing agents, aerosol products, finishing lubricants for textile fibers, air refresheners, animal sprays for farms, cosmetic products, laboratory reagents, and extraction fluids may be mentioned.

The invention is now illustrated by the embodiment described in the example, without wishing to limit the scope of the invention.

### Example

Table 2 shows the properties of a solvent composition based on crude oil (LIAV 75), produced from crude oil by first sulfur depletion, followed by depletion of aromatics, and finally fractionation, and those of the solvent composition of the invention. The solvent composition of the invention is made from palm oil using a method comprising a hydrodeoxygenation of the starting material, followed by isomerization of the product thus obtained, and finally fractionation thereof.

From Table 2 it may be seen that the composition of the invention is substantially different from the solvent composition based on crude oil.

**Table 2.**

| | LIAV 75 | Solvent of biological origin |
|---|---|---|
| | typical | example |
| | %, by weight | %, by weight |
| n-pentane | | 5 |
| C5-isoparaffins | | 6 |
| n-hexane | 35 | 17 |
| C6-isoparaffins | 45 | 22 |
| C6-naphthenes | 20 | <1 |
| n-heptane | | 12 |
| C7-isoparaffins | | 27 |
| C7-naphthenes | | <1 |
| C8-isoparaffins | | 8 |
| C/H ratio | 5.35 | 5.21 |
| iso/n paraffin ratio | | 1.8 |

## Claims

1. Solvent composition, **characterized in that** the solvent composition comprises 25 to 50 % of C₅-C₇ n-alkanes, and 50 to 75 % of C₅-C₇ iso-alkanes, the amount of n-hexanes being 5 to 25 %, respectively by weight, said solvent composition being produced from raw materials of biological origin.

2. Solvent composition according to claim 1, **characterized in that** the solvent composition comprises 30 to 45 % of C₅-C₇ n-alkanes, and 55 to 70 % of C₅-C₇ iso-alkanes, the amount of n-hexane being 15 to 23 %, the amount of n-pentane being 4-8 %, and the amount of n-heptane 11-14 % by weight, said solvent composition being produced from raw materials of biological origin.

3. Solvent composition according to claim 1 or 2, **characterized in that** the solvent composition has a boiling range of 35 to 150 °C, and a density at +15 °C of 660 to 680 kg/m³, said composition comprising no more than 0.01 %, by weight, of aromatic compounds, and less than 2 5, by weight of naphthenic compounds.

4. Solvent composition according to any of the claims 1 to 3, **characterized in that** the solvent composition is a technochemical solvent, an industrial solvents, a fuel, drilling fluids for oil recovery, hydraulic oil, cable oil, heat transfer fluid, agricultural chemical, fuels for heating and lightning purposes, chemical mixture or formulation, cleaning agent, propellant, fuel additive, paint, surface treatment agent, construction material, agent for the production and processing of polymers, solvent or an additive for extraction processes, or a constituent thereof.

5. Solvent composition according to any of the claims 1 to 4, **characterized in that** the solvent composition is a solvent for extraction processes, solvent for road marking paints, or a washing solvent.
